# EUROPEAN PATENT APPLICATION

(11) **EP 4 079 315 A1**
(43) Date of publication of application: **26.10.2022**
(21) Application number: 20901458.8
(22) Date of filing: 18.12.2020
(51) Int. Cl.: A61K 35/57, A23L 33/18, A61K 38/01, A61P 37/06, A61P 37/08

(54) **EGG ALLERGY PREVENTING AGENT OR THE LIKE, AND FOOD COMPOSITION CONTAINING SAME**

(30) Priority: 19.12.2019 JP 2019229144
(71) Applicant: National Center for Child Health and Development, Tokyo 157-8535 (JP); NH Foods Ltd., Osaka-shi, Osaka 530-0001 (JP)
(72) Inventor: AYABE Ayako, Tsukuba-shi, Ibaraki 300-2646 (JP); HASEGAWA Takanori, Tsukuba-shi, Ibaraki 300-2646 (JP); MATSUMOTO Kenji, Tokyo 157-8535 (JP); MORITA Hideaki, Tokyo 157-8535 (JP)
(74) Representative: J A Kemp LLP
(86) International application number: PCT/JP2020/047305
(87) International publication number: WO 2021/125303

(57) **Abstract**

The present invention provides highly effective and safe means capable of preventing or treating an egg allergy in infants. An oral immunotolerance agent or a food composition for preventing or treating an egg allergy according to the present invention contains a degraded product of heat-denatured egg white with an aspartic protease (e.g. pepsin) or a metal protease (e.g. thermolysin).

## Description

### Technical Field

The present invention relates to an oral immunotolerance agent that can be used for prevention and treatment of egg allergy, and a food composition containing the same.

### Background Art

The overwhelming majority of food allergy patients are infants, and in particular, egg (hen's egg) allergy is one of highly prevalent food allergies for infants. Studies have been conducted for many years on a cause of developing food allergies in infants. It has been reported that infants are easily sensitized to food if having atopic dermatitis (Non Patent Literature 1), and it has been pointed out that disorder of the skin barrier function and transdermal antigen entry may be a cause of a food allergy (Non Patent Literature 2). It has also been reported that hen's egg allergens are detected from all of children's beds, and the amount thereof is significantly larger than that of mite allergens (Non Patent Literature 3), and the number of infant egg allergy patients may increase because there are a lot of hen's egg allergens in the environment.

It has been suggested that for preventing egg allergies, a method is effective in which atopic dermatitis is treated to prevent entry of an allergen through an inflamed skin, and then the infant are allowed to eat a small amount of heated whole egg as early as, for example, six months of age (Non Patent Literatures 4 to 6). However, it has been reported that some infants are already sensitized to egg (having anti-egg antibodies generated in the body) at six months of age, and an anaphylactic shock frequently occurs in a clinical test where raw egg powder was ingested (Non Patent Literature 7), and that heated egg (heated egg yolk or heated egg white) is not necessarily safe, and there are many infants who develop allergic reactions (Non Patent Literature 8).

As a more specific method for preventing or treating an egg allergy, for example, the following method has been proposed.

Non Patent Literature 9 discloses that degraded product of non-heated ovalbumin with pepsin is orally given to a mouse for a certain period of time before sensitization to egg white to suppress an anaphylactic shock caused upon administration of egg white. However, the method disclosed in Non Patent Literature 9 merely suggests that an allergy caused by non-heated ovalbumin or non-heated egg white or whole egg (raw egg) containing the ovalbumin can be prevented by oral immunotolerance. Non Patent Literature 9 does not describe a method for preventing allergies caused by heated egg white, particularly ovomucoid which is contained in the egg white and hardly loses allergenicity even when heated.

Patent Literature 1 describes the invention of "a method for production of a purified extract of natural allergens comprising the steps of: a) extracting a natural source of allergens comprising allergenic proteins to form an extract; b) purifying of said extract to remove non-protein components to form a purified extract; and c) denaturating said purified extract to form a purified denaturated extract, said purified denaturated extract comprising proteins, wherein the most abundant (w/w) proteins, forming together at least 60% (w/w) of all proteins, are at least two proteins, and all proteins represent at least 60% (w/w) of the dry weight of the purified denaturated extract" (claim 1), and the invention of "a method for production of a purified extract of natural allergens comprising the steps of: a) hydrolyzing a denaturated allergen to form an allergen hydrolysate; and b) purifying said allergen hydrolysate to remove peptides with a molecular weight above 10,000 Da and below 1,000 Da in order to obtain a purified hydrolysate where 70%, more preferably 80% of the peptides are between 10,000 Da and 1,000 Da" (claim 8). It also sets forth that the "hydrolysis" in the invention is performed using "preferably pepsin, trypsin or chymotrypsin", and the like. In addition, it also sets forth that "prior to hydrolysis, a not denaturated allergen is denaturated to form a denaturated allergen", the denaturating is "performed in the presence of chaotropic agents, reducing agents and mixtures thereof", and the like. Further, it also states that the "purified denaturated extract of natural allergens" and the "purified allergen hydrolysate" which are obtained by the above-mentioned methods are used for "the preparation of a pharmaceutical composition and/or food composition for inducing immunotolerance" and "the induction of immunotolerance is used to cure or prevent of allergic reactions". As the "allergens", "egg allergens" in many foods are also exemplified. However, in Patent Literature 1, an embodiment related to the egg allergens are not disclosed beyond a general statement, and is not disclosed as Example. In particular, using the entire egg white after denaturation by heating, rather than using a purified product obtained by extracting ovalbumin, ovomucoid or the like contained in, for example, egg white, as egg allergens, and effects exhibited thereby are not either disclosed or suggested.

### Citation List

### Patent Literature

Patent Literature 1: Japanese Translation of PCT International Application Publication No. 2010-504278

### Non Patent Literature

Non Patent Literature 1: Flohr et al. J Invest Dermatol. 2014 Feb; 134(2): 345-350. doi: 10. 1038/jid. 2013. 298. Epub 2013 Jul 18.
Non Patent Literature 2: Kubo et al. J Clin Invest. 2012 Feb; 122(2): 440-7. doi: 10. 1172/JCI57416. Epub 2012 Feb 1.
Non Patent Literature 3: Kitagawa H et al. Allergology Int. 2019
Non Patent Literature 4: Fukuie et al. Recommendations on Prevention of Development of Hen's Egg Allergy. 2017
Non Patent Literature 5: Natsume et al. Lancet. 2017 Jan 21; 389(10066): 276-286. doi: 10. 1016/S0140-6736(16)31418-0. Epub 2016 Dec 9.
Non Patent Literature 6: du Toit et al. J Allergy Clin Immunol. 2018 Apr; 141(4): 1343-1353. doi: 10. 1016/j. jaci. 2017. 09. 034. Epub 2017 Oct 31.
Non Patent Literature 7: Matsumoto et al. J Allergy Clin Immunol. 2018 Jun; 141(6): 1997-2001. e3. doi: 10. 1016/j. jaci. 2018. 02. 033. Epub 2018 Mar 6.
Non Patent Literature 8: Ikematsu et al., Japanese Journal of Allergology, 55(5), p.533-541, 2006.
Non Patent Literature 9: Lozano-Ojalvo et al., Clin Exp Allergy, 47(10), p.1342-1354, 2017.

### Summary of Invention

### Technical Problem

An object of the present invention is to provide means capable of preventing or treating an egg allergy in infants, which is a more effective and less hazardous means than conventional means.

### Solution to Problem

The present inventors have found that a product obtained by heat-denatured only egg white of egg and then performing treatment with a specific food proteolytic enzyme (herein, referred to as "PEW" (protease-digested egg white)) can be used as a highly effective and low-risk agent which, when orally ingested, can prevent an egg allergy, particularly an allergy caused by ovomucoid and ovalbumin contained in egg white, and is less likely to causes epicutaneous sensitization even if the skin barrier function is impaired. Thus, the present invention has been completed. In particular, aspartic proteases such as pepsin and metal proteases such as thermolysin (e.g. "Thermoase (R) PC10F") are food proteolytic enzymes which are superior to various other food proteolytic enzymes such as serine protease in stability of effects of preventing an egg allergy, etc. when orally ingested, and are suitable for the present invention.

That is, the present invention encompasses the following items.
[1] An oral immunotolerance agent for preventing or treating an egg allergy, comprising a degraded product of heat-denatured egg white with an aspartic protease or a metal protease.
[2] The oral immunotolerance agent according to item 1, wherein the aspartic protease is pepsin.
[3] The oral immunotolerance agent according to item 1, wherein the metal protease is thermolysin.
[4] A food composition comprising a degraded product of heat-denatured egg white with an aspartic protease or a metal protease.
[5] The food composition according to item 4, for use in prevention or treatment of an egg allergy.
[6] The food composition according to item 4 or 5, wherein the aspartic protease is pepsin.
[7] The food composition according to item 4 or 5, wherein the metal protease is thermolysin.
[8] A method for producing an oral immunotolerance agent for preventing or treating an egg allergy, the method comprising the step of degrading heat-denatured egg white with an aspartic protease or a metal protease.
[9] A method for producing a food composition, the method comprising the step of blending a degraded product of heat-denatured egg white with an aspartic protease or a metal protease into a raw material.
[10] The method for producing a food composition according to item 9, wherein the food composition is for use in prevention or treatment of an egg allergy.
[11] A method for using a degraded product of heat-denatured egg white with an aspartic protease or a metal protease, the method comprising adding the degraded product of heat-denatured egg white with an aspartic protease or a metal protease to food as a component for treating or preventing an egg allergy by oral immunotolerance.

The inventions of [1] to [11] above can be appropriately converted into inventions in other categories. For example, in another aspect, the present invention provides PEW used for preventing or treating an egg allergy, and use of PEW (as an active ingredient) for preventing or treating an egg allergy. In another aspect, the present invention provides use of PEW in production of an oral immunotolerance agent or a food composition for preventing or treating an egg allergy. In another aspect, the present invention provides a method for preventing, treating or suppressing an egg allergy, the method comprising administering (orally ingesting) an effective amount of PEW. Items described herein in connection with [1] to [11] above can also be appropriately converted in line with the inventions described above.

### Advantageous Effects of Invention

An oral immunotolerance agent of the present invention and food containing the same are less likely to cause epicutaneous sensitization due to entry through the skin while being capable of preventing or treating an egg allergy by stably inducing oral immunotolerance when ingested into a subject having a high risk of developing an egg allergy, particularly an infant, and are therefore extremely useful as an agent and food having effectiveness and safety at an unprecedented level. Even in an infant already sensitized to egg, the oral immunotolerance agent of the present invention and food containing the same are expected to be less likely to induce an egg allergy symptom through oral ingestion, and therefore have high safety.

In the present invention, an agent suitable for prevention of an egg allergy, etc. can be obtained by using a "specific food proteolytic enzyme" (aspartic protease, metal protease or the like), and it is considered that the reason for this may be, for example, that a degraded product (PEW) obtained by treating heat-denatured egg white with a "specific food proteolytic enzyme" contains a peptide having a moderate molecular weight, or contains, as a mixture of various peptides generated, one having a moderate molecular weight distribution. That is, it is considered that the peptide (mixture) contained in PEW is an antigen suitable for inducing immunotolerance for Treg cells (regulatory T-cells) (has a T-cell epitope), but is not an antigen producing an antibody for B-cells, can prevent epicutaneous sensitization, is not an antigen for S-IgE (specific IgE), and does not cause an allergy symptom.

On the other hand, although heat-denatured egg white which is not degraded by a food proteolytic enzyme (EW) may be able to induce a certain level of immunotolerance by acting on Treg cells, PEW is superior to EW in effect of suppressing induction of differentiation into Th2-cells (proliferation of Th2-cells) and suppressing production of a cytokine (IL-13 or the like) (see Example 4). EW may cause epicutaneous sensitization by acting on B-cells to produce antibodies, or cause allergy symptoms by acting on S-IgE.

If a food proteolytic enzyme other than the "specific food proteolytic enzyme" is used, there may be cases where even the T-cell epitope necessary for Treg cells is degraded, so that immunotolerance cannot be induced, or the stability of induction of immunotolerance is insufficient.

However, the present invention is not unduly limited by the above-mentioned speculation, and even if, for other reasons, the effects of the present invention are exhibited by using an aspartic protease (e.g. pepsin) or a metal protease (e.g. thermolysin), the present invention is secured by the description herein.

### Brief Description of Drawings

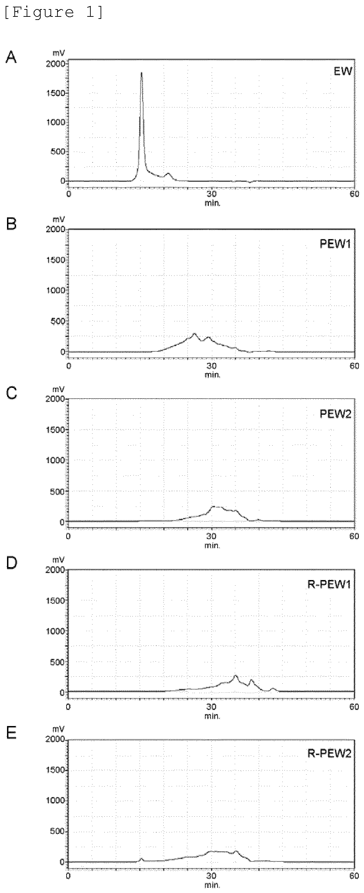

[Figure 1] Figure 1 shows the results of measurements of a molecular weight distribution of proteins (peptides) contained in heat-denatured egg white [A], a molecular weight distribution of peptides contained in pepsin-treated PEW (PEW 1) [B], a molecular weight distribution of peptides contained in Thermoase (R) PC10F-treated PEW (PEW 2) [C], a molecular weight distribution of peptides contained in Protease P "Amano" 3SD-treated PEW (R-PEW 1) [D] and a molecular weight distribution of peptides contained in Protin SD-AY10-treated PEW (R-PEW 2) [E] in Example 3.

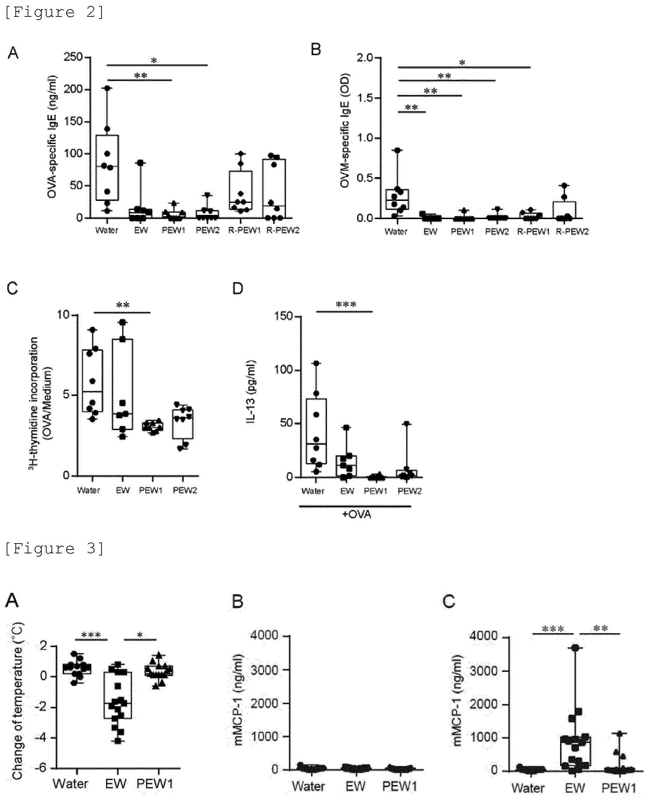

[Figure 2] Figures 2[A] and 2[B] show the results of measurements of the blood levels of IgE specific to OVA and OVM, respectively, in Example 4. Figures 2[C] and 2[D] show the results of measurements of a cell proliferation rate of cells isolated from the mesenteric lymph node and cultured [C] and a concentration of IL-13 in the culture supernatant [D] in Example 3. ***: p < 0.001, **: p < 0.01, *: p < 0.05, non-parametric test: Kruskal-Wallis test, multiple comparison: Dunn's multiple comparisons test, n = 7-8.
[Figure 3] Figure 3 shows the results of measurements of a body temperature change [A] reflecting an anaphylactic shock, and blood levels of mMCP-1 before sample administration [B] and after sample administration [C] in Example 5 using PEW 1 etc. ***: p < 0.001, **: p < 0.01, *: p < 0.05, non-parametric test: Kruskal-Wallis test, multiple comparison: Dunn's multiple comparisons test, n = 13-15 (OVAs IgE in serum: 50 ng/ml or more).

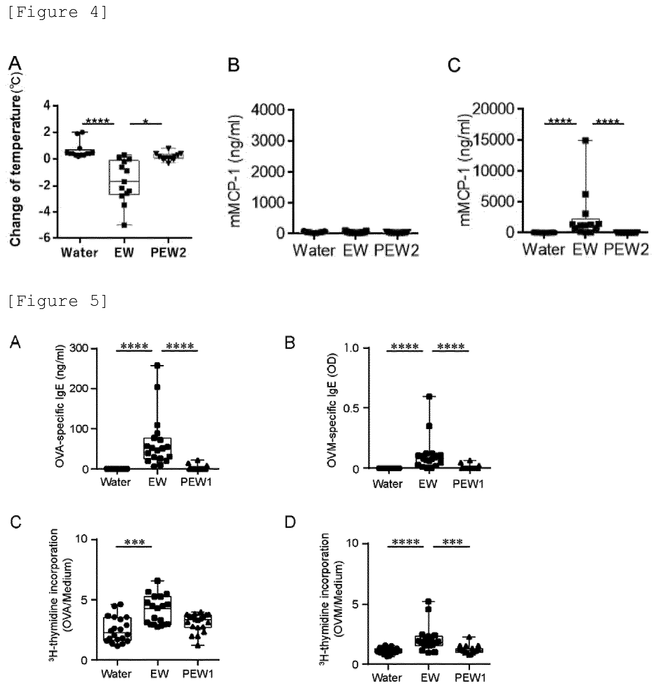

[Figure 4] Figure 4 shows the results of measurements of a body temperature change [A] reflecting an anaphylactic shock induction, and blood levels of mMCP-1 before sample administration [B] and after sample administration [C] in Example 6 using PEW 2 etc. ****: p < 0.0001, *: p < 0.05, non-parametric test: Kruskal-Wallis test, multiple comparison: Dunn's multiple comparisons test, n = 13 (OVAs IgE in serum: 50 ng/ml or more).
[Figure 5] Figures 5[A] and 5[B] show the results of measurements of the blood level of OVA-specific IgE [A] and the blood level of OVM-specific IgE [B] (optical density (OD) of serum diluted by 25 times) in Example 7 using PEW 1 etc. Figures 5[C] and 5[D] show the results of measurements of cell proliferation rates of cells isolated from the spleen and cultured, in OVA stimulation [C] and OVM stimulation [D], respectively, in Example 7. ****: p < 0.0001, ***: p < 0.001, non-parametric test: Kruskal-Wallis test, multiple comparison: Dunn's multiple comparisons test, n = 19-20.

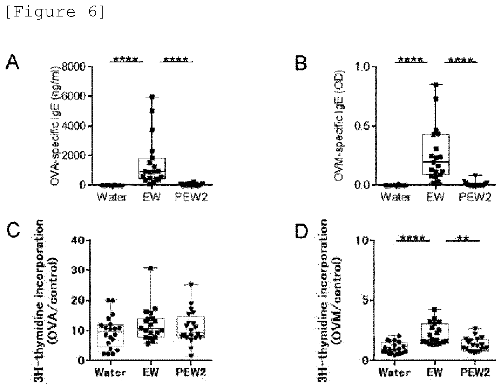

[Figure 6] Figures 6[A] and 6[B] show the results of measurements of the blood level of OVA-specific IgE [A] and the blood level of OVM-specific IgE [B] (optical density (OD) of serum diluted by 25 times) in Example 8 using PEW 2 etc. Figures 6[C] and 6[D] show the results of measurements of cell proliferation rates of cells isolated from the spleen and cultured, in OVA stimulation [C] and OVM stimulation [D], respectively, in Example 8. ****: p < 0.0001, **: p < 0.01, non-parametric test: Kruskal-Wallis test, multiple comparison: Dunn's multiple comparisons test, n = 19-20.

### Description of Embodiments

### -Oral immunotolerance agent-

An oral immunotolerance agent for preventing or treating an egg allergy according to the present invention (herein, referred to as an "oral immunotolerance agent of the present invention") contains a degraded product of heat-denatured egg white with a specific food proteolytic enzyme (referred to as a "specific enzyme" in the present invention) (PEW).

The oral immunotolerance agent of the present invention contains at least PEW as an active ingredient, and may further contain other components as necessary. That is, the oral immunotolerance agent of the present invention may be composed only of PEW, or may further contain other components which are likewise effective for preventing or treating an egg allergy, and various components which are used as food additives.

The oral immunotolerance agent of the present invention may be in a liquid form with the oral immunotolerance agent dissolved in a suitable solvent such as water or a buffer solution, or may be in a powder form with the oral immunotolerance agent prepared in such a liquid form and then subjected to treatment such as powderization etc., or in other solid forms. For example, a powdered oral immunotolerance agent can be produced by powdering PEW by spray-drying, freeze-drying or the like, and blending additives (e.g. excipients, colorants, flavors and sweeteners) at this time as necessary. For example, like a supplement (nutritional supplementary food), an oral immunotolerance agent may be produced with suitable additives used in conformity to a dosage form such as a powder (powdered drug), a granule, a tablet a capsule, other solid dosage forms, a jelly or a solution.

### (PEW)

PEW used in the present invention is a substance prepared by degrading heat-denatured egg white using a specific enzyme. The egg white may be heated for a time at a temperature sufficient to denature a protein (ovalbumin, ovomucoid or the like) contained in the egg white, at least 60 to 80°C, or higher, for example, in boiling water. The egg white may be formed into dry powder for storability, followed by redispersing the powder in water or the like and performing heating in preparation of PEW.

In the present invention, a heat-denatured product of the entire egg white can be targeted to be degraded by a specific enzyme, and it is not necessary that a product obtained by extracting ovalbumin or ovomucoid, which is a main allergen contained in egg white, from egg white (removing other proteins and non-protein components) and then performing heat-denaturation be targeted as in the conventional art. Egg white from chickens contains proteins having allergenicity, such as ovotransferrin (conalbumin) and lysozyme, in addition to ovalbumin and ovomucoid. A degraded product of heat-denatured egg white with a specific enzyme and a degraded product of non-heat-denatured egg white with a specific enzyme can be physically distinguished from each other because the enzymatically cleaved sites varies depending on whether heat-denaturation occurs or not, so that the compositions of peptide mixtures generated are not the same.

The "egg white" may be egg white isolated from whole egg (separated from egg yolk), or egg white contained in whole egg. That is, the degraded product of "heat-denatured egg white" with a specific enzyme may be obtained by enzymatically treating a heat-denatured product of egg white isolated from whole egg, or obtained by enzymatically treating a heat-denatured product of egg white contained in whole egg.

In other words, the oral immunotolerance agent of the present invention "comprising" a degraded product of heat-denatured egg white with a specific enzyme may contain a degraded product of heat-denatured "egg yolk" (protein) with a specific enzyme, which is inevitably generated when a heat-denatured product of egg white contained in whole egg is enzymatically treated, together with a degraded product of heat-denatured "egg white" (protein) with a specific enzyme. The same applies to the food composition of the present invention "comprising" a degraded product of heat-denatured egg white with a specific enzyme, and other related inventions. Regarding the invention of such an embodiment, the (degrade product of) "heat-denatured egg white" (with a specific enzyme) herein can be replaced by the (degraded product of) "heat-denatured whole egg" (with a specific enzyme).

### (Specific enzyme)

As used herein, the "specific enzyme" refers to, among various food proteolytic enzymes (protease, peptidase and the like) derived from various microorganisms, enzymes capable of exhibiting the effects of the present invention, i.e. enzymes capable of preventing an egg allergy when heat-denatured egg white treated with such enzymes is used. Examples of the specific enzyme include "aspartic proteases" (sometimes referred to as "aspartic acid proteases") and "metal proteases" (sometimes referred to as "metalloproteases"). As each of the aspartic protease and the metal protease, various types of proteases are known, and those that are commonly used for food can also be used in the present invention. The specific enzyme may be caused to act on heat-denatured egg white for a suitable time under conditions taking an optimum temperature, optimum pH and the like into consideration.

Specific examples of the "aspartic protease" include pepsin (EC.3.4.23.1-3), chymosin (EC 3.4.23.4) and cathepsin D (EC 3.4.23.5), and it is also possible to use other known aspartic proteases derived from various microorganisms and used for food.

Specific examples of the "metal protease" include thermolysin (EC.3.4.24.27), and it is also possible to use other known metal proteases derived from various microorganisms and used for food. For example, "Thermoase (R) PC10F" (manufactured by Amano Enzyme inc., derived from Geobacillus stearothermophilus) is commercially available as a food protease containing thermolysin.

In addition, serin proteases (trypsin, chymotrypsin, thrombin, plasmin, elastase, subtilisin etc., and products such as "Protease P "Amano" 3SD" and "Protin SD-AY10" used in Examples), and thiol proteases (papain, ficin, bromelain, cathepsin B and the like) are also known as food proteolytic enzymes. However, aspartic proteases such as pepsin and metal proteases such as thermolysin are superior to serin proteases and the like in stability of effect of preventing or treating an egg allergy, for example, the effect of suppressing production of s-IgE, when orally ingested.

It is to be noted that as shown in Example 4 below (Figure 2), the degraded product of heat-denatured egg white (R-PEW 1) using "Protease P "Amano" 3SD" has an action of suppressing production (reducing the blood level) of an OVM (ovomucoid)-specific IgE antibody. Ovomucoid is a protein which is less likely to be denatured by heating and has higher allergenicity over ovalbumin. Therefore, in an embodiment of the present invention, it is also possible to use "Protease P "Amano" 3SD" as a specific enzyme.

### (Egg)

The "egg" in the present invention is typically egg from chickens (hen's egg). That is, in a typical embodiment of the present invention, heat-denatured egg white for use in production of the oral immunotolerance agent is egg white of hen's egg, and the egg allergy to be prevented by the present invention is a hen's egg allergy. However, the egg in the present invention is not limited to hen's egg, and there may be a possibility that using egg (heat-denatured egg white) containing a protein highly homologous with an allergen protein of hen's egg, such as egg from quails, ducks and other edible birds, the present invention can be carried out to prevent or treat allergies to the egg.

### (Preparation method)

When pepsin is used as a specific enzyme, PEW can be prepared in accordance with, for example, the following steps. For aspartic proteases other than pepsin, similar steps modified as necessary can be adopted.
(1) Egg white formed into dry powder in advance is diluted with water to prepare an egg white aqueous solution at a suitable concentration (e.g. 20 mg/mL).
(2) The egg white aqueous solution is heated at a suitable temperature (e.g. in boiling water) for a suitable time (e.g. about 10 minutes) for denaturation of egg white.
(3) The pH of the heat-denatured egg white aqueous solution is adjusted to be within a range suitable for pepsin (e.g. to 1.5) by adding an acid (e.g. HCl).
(4) An appropriate amount of pepsin (e.g. 2 wt% with respect to the heat-denatured egg white aqueous solution) is added to the heat-denatured egg white aqueous solution.
(5) The heat-denatured egg white aqueous solution is maintained at a suitable temperature (e.g. 37°C) for a suitable time (e.g. 180 minutes) for application of pepsin.
(6) The pH of the pepsin-treated heat-denatured egg white aqueous solution is adjusted to a near-neutral pH by adding an alkali (e.g. NaOH).
(7) The pepsin-treated heat-denatured egg white aqueous solution is heated at a suitable temperature (e.g. in boiling water) for a suitable time (e.g. about 15 minutes) to inactivate the enzyme.

When "Thermoase (R) PC10F" is used as a specific enzyme, PEW can be prepared in accordance with, for example, the following steps. For metal proteases other than "Thermoase (R) PC10F" (thermolysin), similar steps modified as necessary can be adopted.
(1) Egg white formed into dry powder in advance is diluted with water to prepare an egg white aqueous solution at a suitable concentration (e.g. 20 mg/mL).
(2) The egg white aqueous solution is heated at a suitable temperature (e.g. in boiling water) for a suitable time (e.g. about 10 minutes) for denaturation of egg white.
(3) The pH of the heat-denatured egg white aqueous solution is adjusted to be within a suitable range for "Thermoase (R) PC10F" (e.g. to 8.0) by adding an alkali (e.g. NaOH).
(4) An appropriate amount of "Thermoase (R) PC10F" (e.g. 5 wt% with respect to the heat-denatured egg white aqueous solution) is added to the heat-denatured egg white aqueous solution.
(5) The heat-denatured egg white aqueous solution is held at a suitable temperature (e.g. 65°C) for a suitable time (e.g. 180 minutes) for application of "Thermoase (R) PC10F".
(6) The pH of the "Thermoase (R) PC10F"-treated heat-denatured egg white aqueous solution is adjusted to a near-neutral pH by adding an acid if necessary.
(7) The "Thermoase (R) PC10F"-treated heat-denatured egg white aqueous solution is heated at a suitable temperature (e.g. in boiling water) for a suitable time (e.g. about 15 minutes) to inactivate the enzyme.

PEW contains degraded products by specific enzyme of egg white-derived proteins such as ovalbumin and ovomucoid (peptide mixture), and other components. Depending on an embodiment of treatment using a specific enzyme (e.g. type of specific enzyme, treatment time, treatment temperature and other treatment conditions), the manner of degrading the protein varies, and the composition and the molecular weight distribution of the resulting peptide mixture may vary, but such variations are acceptable as long as the effects of the present invention are exhibited. The molecular weight distribution of the peptide mixture can be measured under suitable conditions using a known conventional method such as size exclusion chromatography (SEC). Standard hen's egg contains water at about 90% and protein at about 10%, and the protein contains ovalbumin at about 54%, ovomucoid at about 11% and ovotransferrin at about 12%.

### (Use)

The oral immunotolerance agent of the present invention can be itself ingested alone as food like a supplement, or can be added in an appropriate amount to other food and ingested. The oral immunotolerance agent of the present invention may be prepared in advance, and blended into a food composition as a part of a raw material for producing the food composition of the present invention, particularly as a component for inducing oral immunotolerance.

However, the use of the oral immunotolerance agent of the present invention is not limited to those described above, and the oral immunotolerance agent can be used for various purposes related to the effect of preventing or treating an egg allergy. For example, the embodiment in which a degraded product of heat-denatured egg white with a specific enzyme is used *in vitro* for the purpose of, for example, analyzing interaction with a cell (e.g. Treg cell, B-cell, dendritic cell or another immune-related cell) or an antibody (s-IgE) which may be related to oral immunotolerance to an egg allergy is also an embodiment of the oral immunotolerance agent of the present invention.

The oral immunotolerance agent of the present invention can be ingested into (administered to) a subject desiring to have oral immunotolerance induced for preventing or treating an egg allergy. Such a subject may be a human, or a non-human mammal (e.g. mouse, rat, rabbit, guinea pig, sand rat, hamster, ferret, dog, miniature pig, monkey, bovine, horse or sheep). When the subject is a human, it is preferable to ingest the oral immunotolerance agent into an infant, e.g. an infant between the age of six months when ingestion of baby food is started and the age of one year, or below the elementary school age when an egg allergy is likely to be developed. When an egg allergy is prevented, the oral immunotolerance agent of the present invention may be orally ingested into a subject that has not been sensitized to an egg white-derived allergen, typically ovalbumin (OVA), ovomucoid (OVM) or the like, and when an egg allergy is treated, the oral immunotolerance agent of the present invention may be orally ingested into a subject that has been sensitized to such an allergen.

The approximate ingestion dose per day or the approximate ingestion dose per serving and the ingestion frequency per day of the oral immunotolerance agent of the present invention, and the duration (the number of days) of ingestion can be appropriately set on the basis of the embodiment of oral immunotolerance of the present invention, the age, body weight and sex of a subject that is subjected to oral ingestion (oral administration) thereof, an indication or attribute related to an egg allergy (whether the subject is sensitized to an egg white-derived allergen, whether atopic dermatitis is developed, or the like), an expected degree of the effect of preventing or treating an egg allergy, the results of nonclinical or clinical tests on a human or a non-human mammal as a subject, and the like.

The effect of preventing an egg allergy by the oral immunotolerance agent of the present invention can be evaluated by, for example, the following method.
(A1) The oral immunotolerance agent of the present invention is ingested into (administered to) a subject (e.g. mouse) for a certain period of time.
(A2) Ovalbumin (OVA) which is a main allergen contained in egg white, or OVA and ovomucoid (OVM) are intraperitoneally administered (injected) to a subject, together with an adjuvant (e.g. aluminum salt) as necessary, to cause sensitization. This step may be carried out multiple times with intervals over a period of time as necessary.
(A3) Blood is collected from the subject, and the concentration of an antibody (IgE) specific to OVA or OVM is measured. Examination is conducted on whether or not there is a statistically significant difference in antibody concentration in comparison with a control group in which a substance other than the oral immunotolerance agent of the present invention (e.g. water or egg white (EW) that is not degraded by a protein hydrolysis enzyme) is orally ingested in (A1) above. For example, when the antibody concentration is statistically significantly low in comparison with a control group in which water is orally ingested, it is possible to evaluate that by orally ingesting the oral immunotolerance agent of the present invention in advance, production of an IgE antibody to an egg white allergen can be suppressed, i.e. an effect of being capable of preventing an egg allergy is exhibited.

The safety of the oral immunotolerance agent of the present invention can be evaluated by, for example, the following method from the viewpoint of a risk of causing an anaphylactic shock and a possibility of epicutaneous sensitization.
(B1) Similarly to (A2) above, OVA, or OVA and OVM are intraperitoneally administered (injected) to a subject, together with an adjuvant as necessary, to cause sensitization.
(B2) The oral immunotolerance agent of the present invention is intraperitoneally administered to the subject after a certain period of time from the sensitization.
(B3) A change in body temperature of the subject before and after the administration (e.g. after 30 minutes) is measured. Examination is conducted on whether or not there is a statistically significant difference in body temperature change in comparison with a control group to which a substance other than the oral immunotolerance agent of the present invention (e.g. water or EW) is administered in (B2) above. For example, when there is no statistically significant difference in body temperature change in comparison with a control group in which water is administered, it is possible to evaluate that the oral immunotolerance agent of the present invention does not cause a body temperature change (decrease) due to anaphylaxis, i.e. the oral immunotolerance agent has a low risk.
(C1) The oral immunotolerance agent of the present invention is applied on a dermal inflammation site of the subject (e.g. mouse) for a certain period of time. This step may be carried out multiple times with intervals over a period of time as necessary.
(C2) Blood is collected from the subject, and the concentration of an antibody (IgE) specific to OVA or OVM is measured. Examination is conducted on whether or not there is a statistically significant difference in antibody concentration in comparison with a control group in which a substance other than the oral immunotolerance agent of the present invention (e.g. water or EW) is applied in (C1) above. For example, when there is no statistically significant difference in antibody concentration in comparison with a control group in which water is administered, it is possible to evaluate that the oral immunotolerance agent of the present invention hardly produces an antibody to OVA or OVM, i.e. the oral immunotolerance agent is less likely to cause epicutaneous sensitization.

For more specific embodiments of the various evaluation methods described above, it is possible to refer to Examples herein. When through these evaluation methods, whether or not there is a statistically significant difference, a level of significance, and the like are considered, it is possible to pursue oral immunotolerance agents (or food compositions containing the agents) of more preferred embodiments, preferred conditions for treatment with, for example, a specific enzyme, the manner in which the oral immunotolerance agent is ingested into a human or a non-human mammal, i.e. the dose for each ingestion and the duration, and the like.

### -Food composition-

The food composition of the present invention comprises at least a degraded product of heat-denatured egg white with a specific enzyme (PEW), e.g. with an aspartic protease (pepsin or the like) or a metal protease ("Thermoase (R) PC10F" or the like), that is the oral immunotolerance agent of the present invention, and further at least one food raw material, food additives and other components which are suitable for embodiments of the food composition.

The food composition of the present invention is typically a food composition (invention for use in food) for preventing or treating an egg allergy. Even if such a use purpose is not highlighted, the food composition of the present invention can be ingested as a low-risk food composition into a subject that may develop an egg allergy (e.g. infant), or a subject that develops an egg allergy and/or atopic dermatitis.

The type of the food composition of the present invention is not particularly limited, and can be produced as one that is the same type as common food. Examples of the food include soft drinks (juice, coffee drinks, tea drinks, mineral water, sports drinks and the like), milk drinks, soymilk drinks, fermented milk (yogurt), lactic acid bacteria beverages, cocoa, drinks prepared from instant powder, jelly, pudding, confectioneries (biscuit, cookie, chocolate and the like), bread, cereal, prepared food, and seasoning agents (mayonnaise, dressing and the like). The food composition of the present invention is preferably in the form of baby food suitable for ingestion by infants, and for example, it is preferable that various kinds of food exemplified above be produced as baby food of wet type (liquid or semisolid food such as retort-packed food and canned food) or baby food of dry type (powdered, granular, flaky and solid food which are eaten with water or hot water added to restore an original shape).

The content of PEW of the food composition of the present invention (oral immunotolerance agent of the present invention) is not particularly limited as long as an effect of preventing or treating an egg allergy is exhibited by ingesting the food composition. Such a content can be set with consideration given to the amount of PEW (oral immunotolerance agent as an active ingredient) per serving or per day, the weight of the ingested food composition and the like, and also with consideration given to the form of the food composition, the production method and the like as appropriate. The amount of PEW per serving or per day in relation to the food composition of the present invention is as described above in relation to the oral immunotolerance agent of the present invention.

The food composition of the present invention can be produced by the same method as those for conventional food compositions except for further comprising the step of blending PEW (oral immunotolerance agent of the present invention) as one of raw materials. PEW can be prepared in advance in the above-described manner, and stored until production of the food composition.

It can be directly or indirectly indicated that the food composition of the present invention is used for treating or preventing an egg allergy. Examples of the direct indication include written indications on entities such as products themselves, packages, containers, labels and tags, and examples of the indirect indication include advertising via sites or means such as websites, store fronts, exhibitions, signs, signboards, newspapers, magazines, televisions, radios, mailed materials and e-mails.

### Examples

### [Example 1] Preparation of pepsin-treated PEW (PEW 1)

Dry powder of egg white of hen's egg was added to pure water to prepare an egg white aqueous solution at a concentration of 20 mg/mL. The egg white aqueous solution was heated in boiling water, and held for 10 minutes. To the obtained heat-denatured egg white aqueous solution, hydrochloric acid was added to adjust the pH to 1.5. To the heat-denatured egg white aqueous solution, 2 wt% pepsin (pepsin from porcine gastric mucosa powder ≥ 250 units/mg solid, SIGMA) was added, and the mixture was held at 37°C for 180 minutes. To the obtained pepsin-treated heat-denatured egg white aqueous solution, sodium hydroxide was added to perform neutralization, and the mixture was then heated in boiling water for 15 minutes to inactivate the enzyme. The obtained aqueous solution was defined as an aqueous solution of PEW 1.

### [Example 2] Preparation of PEW (PEW 2) using "Thermoase (R) PC10F"

Dry powder of egg white of hen's egg was added to pure water to prepare an egg white aqueous solution at a concentration of 20 mg/mL. The egg white aqueous solution was heated in boiling water, and held for 10 minutes. To the obtained heat-denatured egg white aqueous solution, sodium hydroxide was added to adjust the pH to 8.0. To the heat-denatured egg white aqueous solution, 5 wt% "Thermoase (R) PC10F" (Amano Enzyme inc., hereinafter, abbreviated as "PC10F") was added, and the mixture was held at 65°C for 180 minutes, and then heated in boiling water for 15 minutes to deactivate the enzyme. The obtained PC10F-treated heat-denatured egg white aqueous solution was defined as an aqueous solution of PEW 2.

### [Reference Example 1] Preparation of degraded product of heat-denatured egg white (R-PEW 1) using "Protease P "Amano" 3SD"

Dry powder of egg white of hen's egg was added to pure water to prepare an egg white aqueous solution at a concentration of 20 mg/mL. The egg white aqueous solution was heated in boiling water, and held for 10 minutes. To the obtained heat-denatured egg white aqueous solution, sodium hydroxide was added to adjust the pH to 8.0. To the heat-denatured egg white aqueous solution, 6.7 wt% "Protease P "Amano" 3SD" (Amano Enzyme inc., hereinafter, abbreviated as "P3SD") was added, and the mixture was held at 40°C for 180 minutes, and then heated in boiling water for 15 minutes to deactivate the enzyme. The obtained P3SD-treated heat-denatured egg white aqueous solution was defined as an aqueous solution of R-PEW 1.

### [Reference Example 2] Preparation of degraded product of heat-denatured egg white (R-PEW 2) using "Protin SD-AY10"

Dry powder of egg white of hen's egg was added to pure water to prepare an egg white aqueous solution at a concentration of 20 mg/mL. The egg white aqueous solution was heated in boiling water, and held for 10 minutes. To the obtained heat-denatured egg white aqueous solution, sodium hydroxide was added to adjust the pH to 9.0. To the heat-denatured egg white aqueous solution, 8.3 wt% "Protin SD-AY10" (Amano Enzyme inc., hereinafter, abbreviated as "AY") was added, and the mixture was held at 60°C for 180 minutes, and then heated in boiling water for 15 minutes to deactivate the enzyme. The obtained AY-treated heat-denatured egg white aqueous solution was defined as an aqueous solution of R-PEW 2.

[Example 3] The molecular weight distribution of peptides contained in heat-denatured egg white, and the molecular weight distributions of peptides contained in PEW 1, PEW 2, R-PEW 1 and R-PEW 2 prepared in Examples 1 and 2 and Reference Examples 1 and 2 were measured using HPLC manufactured by Shimadzu Corporation (column: Superdex peptide 10/300 GL, eluent: 0.1% TFA, 30% CAN, light absorption wavelength: 214 nm). Figure 1 shows the results.

### [Example 4] Comparison of preventive effects for hen's egg allergy of PEW using various enzymes

Pepsin-treated PEW 1 and "Thermoase (R) PC10F"-treated PEW 2 prepared in Examples 1 and 2 and "Protease P "Amano" 3SD"-treated R-PEW 1 and "Protin SD-AY10"-treated R-PEW 2 prepared in Reference Examples 1 and 2 were each orally administered in an amount of 2 mg to a test animal (BALB/c strain, female, 3 to 4 weeks old) for 5 days (day -18 to - 14). One week later, PEWs 1 and 2 and R-PEWs 1 and 2 were each orally administered again in an amount of 2 mg to the test animal for 5 days (day -11 to -7). One week later (day 0), 100 µg of each of ovalbumin (OVA) and ovomucoid (OVM) was intraperitoneally injected to the test animal together with an aluminum salt as an adjuvant. Two weeks later (day 14), 100 µg of each of OVA and OVM was intraperitoneally injected to the test animal again together with an aluminum salt. 13 days later (day 27), blood was collected from the test animal, and the blood level of IgE specific to OVA or OVM was measured. Except that water or 2 mg of heat-denatured egg white (EW) was orally administered instead of various enzyme-treated heat-denatured egg white aqueous solutions, the same procedure as described above was carried out to provide a control group.

Figures 2[A] and 2[B] show the results. It is considered that for both OVA-specific IgE and OVM-specific IgE, oral ingestion of any of PEW 1 of Example 1 and PEW 2 enables suppression of production of the IgE, i.e. prevention of an egg allergy, with higher reliability over heat-denatured egg white (EW in the figure). On the other hand, there was no significant difference in comparison with the water group in blood level of the OVA-specific IgE antibody of each of R-PEW 1 and R-PEW 2. It is suggested that the degraded product of heat-denatured egg white is not necessarily useful as an oral immunotolerance agent even when treated with a food proteolytic enzyme, and a degraded product of heat-denatured egg white useful as an oral immunotolerance agent can be obtained only by selecting and using a specific proteolytic enzyme such as pepsin or "Thermoase (R) PC10F".

Furthermore, one week after the blood collection (day 34), the test animal was dissected to take the mesenteric lymph node, and cells contained therein were isolated. OVA was added to a culture medium, the cells were cultured for 72 hours, the culture supernatant was then collected, and the amount of cytokine IL-13 contained therein was determined by an ELISA method. The cell proliferation rate was measured by an assay in which ³H thymidine was added to the culture medium and incorporated into the cells.

Figures 2[C] and 2[D] show the results. In PEW 1 of Example 1, OVA antigen-specific cell proliferation [C] and antigen stimulation-specific IL-13 production [D] unique to a sensitized mouse were not observed. From this, it is considered that differentiation into Th-2 cells by sensitization treatment can be suppressed by oral administration of PEW 1.

### [Example 5] Evaluation on risk of anaphylactic shock by PEW 1

100 µg of each of ovalbumin (OVA) and ovomucoid (OVM) was intraperitoneally injected to a test animal (BALB/c strain, female, 6 weeks old) together with an aluminum salt as an adjuvant to sensitize the test animal to these allergens. Two weeks later (day 14), 100 µg of each of OVA and OVM was intraperitoneally injected to the test animal again together with an aluminum salt to similarly sensitize the test animal. 14 days later (day 28), water, heat-denatured egg white (EW) and PEW 1 of Example 1 were intraperitoneally administered in an amount of 200 mg/kg to the test animal (intraperitoneal challenge). The body temperature was measured during the administration and 30 minutes after the administration, and a body temperature change therebetween was calculated. Two days before the intraperitoneal challenge (day 26) and after the test (day 28), blood was collected from the test animal, and the blood level of mMCP-1 (mouse mast cell protease 1) which is a marker produced upon activation of a mast cell was measured.

Figure 3 shows the results. [A] reveals that when the subject of administration is sensitized by OVA and OVM, administration of heat-denatured egg white (EW in the figure) caused a body temperature change (decrease) due to an anaphylactic shock, and PEW 1 of Example 1 did not cause such a body temperature change. [B] reveals that before the intraperitoneal administration, there was no difference in blood level of MCP-1 among the groups, and [C] reveals that the blood level of MCP-1 after the administration is significantly lower for PEW 1 of Example 1 than for the heat-denatured egg white (EW in the figure). From these results, it is considered that the pepsin-treated product of heat-denatured egg white (PEW 1) has less risk of inducing an anaphylactic shock than the heat-denatured egg white (EW).

### [Example 6] Evaluation on risk of anaphylactic shock by PEW 2

A test animal (BALB/c strain, female, 6 weeks old) was sensitized to OVA and OVM in accordance with the same procedure as in Example 5. 14 days after the second intraperitoneal injection (day 28), water, heat-denatured egg white (EW) and PEW 2 of Example 2 were intraperitoneally administered in an amount of 200 mg/kg to the test animal (intraperitoneal challenge). Two days before the intraperitoneal challenge (day 26) and after the test (day 28), blood was collected from the test animal, and the blood level of mMCP-1 (mouse mast cell protease 1) which is a marker produced upon activation of a mast cell was measured.

Figure 4 shows the results. [A] reveals that when a subject under administration is sensitized by OVA and OVM, administration of heat-denatured egg white (EW in the figure) caused a body temperature change (decrease) due to an anaphylactic shock, and PEW 2 of Example 2 did not cause such a body temperature change. [B] reveals that before the intraperitoneal administration, there was no difference in blood level of MCP-1 among the groups, and [C] reveals that the blood level of MCP-1 after the administration is significantly lower for PEW 2 of Example 2 than for the heat-denatured egg white (EW in the figure). From these results, it is considered that the "Thermoase (R) PC10F"-treated product of heat-denatured egg white (PEW 2) has less risk of inducing an anaphylactic shock than the heat-denatured egg white (EW).

### [Example 7] Evaluation on possibility of epicutaneous sensitization to PEW 1

A test animal (BALB/c strain, female, 6 weeks old) was subjected to epicutaneous sensitization in accordance with the following procedure. The operation of shearing off the hair on the back of the test animal, removing the hair with cream and then attaching and peeling a tape was carried out three times, and water, 4 mg of heat-denatured egg white (EW) or 4 mg of PEW 1 of Example 1 was then applied to the back. The test animal was bandaged, held for 3 days, then unbandaged and held for 7 days. This cycle was repeated three times (day 0 to day 9, day 10 to day 19 and day 20 to day 29). On day 31, blood is collected, and the blood levels of the OVA-specific IgE antibody and the OVM-specific IgE antibody were measured. Further, on day 32, the test animal was dissected to take the spleen, and cells contained therein were isolated. OVA or OVM was added to a culture medium, the cells were cultured for 72 hours, and the cell proliferation rate was then measured by an assay in which ³H thymidine was added to the culture medium and incorporated into the cells.

Figure 5 shows the results. Both the production amounts of the OVA-specific IgE antibody [A] and the OVM-specific IgE antibody [B] in PEW 1 of Example 1 were significantly lower than those in heat-denatured egg white (EW in the figure), and so little that there was no significant difference in comparison with the control (Water in the figure). From these results, it is considered that the pepsin-treated degraded product of heat-denatured egg white (PEW 1) has little risk of causing epicutaneous sensitization, or is at least less likely to cause epicutaneous sensitization than heat-denatured egg white (EW). Furthermore, for the OVA stimulation [C], PEW 1 of Example 1 shows a lower value of the antigen-specific cell proliferation rate in comparison with heat-denatured egg white (EW in the figure) although there is no statistically significant difference in antigen-specific cell proliferation rate between the PEW 1 and the heat-denatured egg white, and for the OVM stimulation [D], antigen-specific cell proliferation is more suppressed in PEW 1 of Example 1 than in heat-denatured egg white (EW in the figure). Thus, PEW 1 of Example 1 is considered to be excellent because T-cells which are proliferated specifically to OVA and OVM are less likely to differentiate even when epicutaneous sensitization treatment is performed.

### [Example 8] Evaluation on possibility of epicutaneous sensitization to PEW 2

A test animal (BALB/c strain, female, 6 weeks old) was subjected to epicutaneous sensitization in the same manner as in Example 7 except that PEW 2 of Example 2 was used instead of PEW 1 of Example 1. Thereafter, the blood levels of the OVA-specific IgE antibody and the OVM-specific IgE antibody, and the proliferation rate of spleen cells were measured.

Figure 6 shows the results. Both the production amounts of the OVA-specific IgE antibody [A] and the OVM-specific IgE antibody [B] in PEW 2 of Example 2 were significantly lower than those in heat-denatured egg white (EW in the figure), and so little that there was no significant difference in comparison with the control (Water in the figure). From these results, it is considered that the "Thermoase (R) PC10F"-treated degraded product of heat-denatured egg white (PEW 2) has little risk of causing epicutaneous sensitization, or is at least less likely to cause epicutaneous sensitization than heat-denatured egg white (EW). Furthermore, for the OVA stimulation [C], PEW 1 of Example 1 shows a lower value of the antigen-specific cell proliferation rate in comparison with heat-denatured egg white (EW in the figure) although there is no statistically significant difference in antigen-specific cell proliferation rate between the PEW 1 and the heat-denatured egg white, and for the OVM stimulation [D], antigen-specific cell proliferation is more suppressed in PEW 2 of Example 2 than in heat-denatured egg white (EW in the figure). Thus, PEW 2 of Example 2 is considered to be excellent because T-cells which are proliferated specifically to OVA and OVM are less likely to differentiate even when epicutaneous sensitization treatment is performed.

## Claims

1. An oral immunotolerance agent for preventing or treating an egg allergy, comprising a degraded product of heat-denatured egg white with an aspartic protease or a metal protease.

2. The oral immunotolerance agent according to claim 1, wherein the aspartic protease is pepsin.

3. The oral immunotolerance agent according to claim 1, wherein the metal protease is thermolysin.

4. A food composition comprising a degraded product of heat-denatured egg white with an aspartic protease or a metal protease.

5. The food composition according to claim 4, for use in prevention or treatment of an egg allergy.

6. The food composition according to claim 4 or 5, wherein the aspartic protease is pepsin.

7. The food composition according to claim 4 or 5, wherein the metal protease is thermolysin.

8. A method for producing an oral immunotolerance agent for preventing or treating an egg allergy, the method comprising the step of degrading heat-denatured egg white with an aspartic protease or a metal protease.

9. A method for producing a food composition, the method comprising the step of blending a degraded product of heat-denatured egg white with an aspartic protease or a metal protease into a raw material.

10. The method for producing a food composition according to claim 9, wherein the food composition is for use in prevention or treatment of an egg allergy.

11. A method for using a degraded product of heat-denatured egg white with an aspartic protease or a metal protease, the method comprising adding the degraded product of heat-denatured egg white with an aspartic protease or a metal protease to food as a component for treating or preventing an egg allergy by oral immunotolerance.
